# FASCICULE DE BREVET EUROPEEN

(11) **EP 4 142 632 B1**
(45) Date de publication et mention de la délivrance du brevet: **13.08.2025**
(21) Numéro de dépôt: 21721135.8
(22) Date de dépôt: 30.04.2021
(51) Int. Cl.: A61B 34/00, A61B 34/37, A61F 9/007, A61B 90/00

(54) **INTERFACE DE PILOTAGE ET SYSTÈME ROBOTISÉ COMPRENANT UNE TELLE INTERFACE DE PILOTAGE**
STEUERSCHNITTSTELLE UND ROBOTERSYSTEM MIT SOLCH EINER STEUERSCHNITTSTELLE
CONTROL INTERFACE AND ROBOTIC SYSTEM COMPRISING SUCH A CONTROL INTERFACE

(30) Priorité: 30.04.2020 FR 2004321
(43) Date de publication de la demande: 08.03.2023
(73) Titulaire: Acusurgical, 34000 Montpellier (FR); CENTRE NATIONAL DE LA RECHERCHE SCIENTIFIQUE, 75016 Paris (FR); UNIVERSITE DE MONTPELLIER, 34090 Montpellier (FR)
(72) Inventeur: SPUHLER, Christoph, 34090 Montpellier (FR); HADDAB, Yassine, 34080 Montpellier (FR); POIGNET, Philippe, 34150 Gignac (FR); MOREL, Antoine, 34090 Montpellier (FR); SANCHEZ, Alonso, 34990 Juvignac (FR)
(74) Mandataire: Gevers & Orès
(86) Numéro de dépôt international: PCT/EP2021/061439
(87) Numéro de publication internationale: WO 2021/219863

(56) Documents cités:
- US-A1- 2012 053 701
- US-A1- 2018 250 086
- US-A1- 2019 333 635
- US-B2- 10 271 914
- US-B2- 9 579 088

## Description

### Domaine technique de l'invention

L'invention a trait au domaine des plateformes robotisées permettant d'effectuer des opérations de chirurgie vitréo-rétinienne.

### Arrière-plan technique

Les opérations de chirurgie vitréo-rétinienne requièrent l'insertion d'instruments chirurgicaux dans l'œil du patient. Classiquement, les instruments chirurgicaux sont insérés au niveau de la partie antérieure de l'œil du patient au moyen d'une tige cylindrique creuse et pointue appelée trocart. Grâce à son insertion dans le trocart, l'instrument traverse la sclère de l'oeil, puis le vitré avant d'atteindre la partie postérieure de l'œil où se situe la rétine. Le chirurgien ne peut pas se passer du trocart. En effet, ce dernier contient une valve qui empêche les fuites de fluides tout en permettant de faire varier aisément la profondeur et l'orientation de l'outil dans l'œil. Il constitue donc un point de passage autour duquel le chirurgien effectue des mouvements avec l'instrument pour le positionner de manière appropriée sur la rétine.

Les chirurgies vitréo-rétiniennes demandent d'intervenir sur des structures dont la taille peut ne faire que quelques dizaines de micromètres. Les chirurgiens doivent pour cela faire preuve d'une très grande dextérité, alors qu'eux même se trouvent souvent dans des positions inconfortables. Certaines opérations demeurent même parfois irréalisables, nécessitant des capacités au-delà des limites humaines.

Le document US 10,271,914 B2 divulgue un système robotisé 100 pour la chirurgie vitréo-rétinienne. Le système robotisé 100 comprend une plateforme robotisée 126, à laquelle est fixé un instrument chirurgical 114, et un dispositif 118 haptique servant à manipuler la plateforme robotisée 126. La plateforme robotisée 126 comprend un effecteur terminal permettant de contrôler l'instrument selon quatre ou six degrés de liberté, l'instrument étant alors capable au minimum d'effectuer des mouvements de translation et des mouvements de rotation autour de son axe. Le dispositif 118 haptique fournit un retour de position, ce qui permet au chirurgien de contrôler à distance les mouvements de la plateforme robotisée 126 en utilisant un stylet 122. Aucune contrainte n'existe sur les mouvements qui peuvent être réalisés avec le stylet 122. Par conséquent, puisqu' en réalité l'instrument 114 est contraint par son passage dans le trocart, certaines actions effectuées par le chirurgien avec le stylet 122 sont irréalisables.

Le document US 6,063,095 B divulgue un système robotisé 10 comprenant une plateforme robotisée consistant en un ensemble de bras robotiques 26 auxquels doivent être fixés des instruments chirurgicaux et un système de pilotage de la plateforme robotisée. Le système de pilotage comprend deux organes de commande 50, 52 permettant de contrôler à distance les mouvements effectués par les bras robotisés 26. Les organes de commande 50, 52 peuvent être montés au choix sur une armoire portable 54 ou un support 900. Dans les deux cas, un jeu de joints JM1-JM5 autorise des mouvements de translation, de rotation des organes de commandes 50, 52 autour de leur axe respectif ainsi que des changements d'orientation de ceux-ci. Chaque joint est associé à un capteur de position (e.g. potentiomètre) afin de déterminer la position finale des organes de commande 50, 52. Ce document ne divulgue pas précisément comment les données des capteurs de position sont traitées pour commander les bras robotisés 26. En tout état de cause, les organes de commande 50, 52 ne sont liés à aucun dispositif haptique.

Pour utiliser ces systèmes, le chirurgien peut devoir se former à des manipulations de bras articulés, très différentes par nature de celles qu'il effectue avec un instrument chirurgical. En outre, le passage d'une opération usuelle à une opération assistée au moyen d'un système robotisé peut se révéler troublant pour le chirurgien qui doit se réadapter à chaque fois qu'il change de mode opératoire.

### Résumé de l'invention

L'invention vise à surmonter les problèmes précités et propose à cet effet une interface de pilotage d'un système robotisé pour la chirurgie vitréo-rétinienne comprenant un dispositif haptique muni d'une chaîne articulée ayant une extrémité libre, caractérisée en ce qu'elle comprend un dispositif de guidage comportant :
- une surface fixe,
- un moyen de guidage monté sur la surface fixe par une liaison rotule,
- une tige montée sur ledit moyen de guidage par une liaison glissière d'axe correspondant à celui de la tige, ladite tige comportant une première extrémité montée fixe sur l'extrémité libre du dispositif haptique et une deuxième extrémité sur laquelle est destiné à être monté un organe de préhension.

Lorsque le chirurgien utilise l'interface de pilotage selon l'invention, celui-ci effectue les mêmes manipulations que s'il manipulait l'instrument chirurgical lui-même. En effet, lorsque le chirurgien manipule l'organe préhenseur, les actions exercées sur celui-ci sont reproduites en bout de chaîne par l'instrument chirurgical.

À cet égard, l'agencement de la tige par rapport à la surface fixe joue un rôle central dans la transmission des mouvements effectués au moyen de l'organe de préhension. La tige étant montée sur le moyen de guidage par une liaison glissière et le moyen de guidage lui-même étant monté sur la surface fixe par une liaison rotule, la tige est contrainte de la même manière qu'un instrument est contraint par son passage dans le trocart. C'est ainsi que son enfoncement et son orientation peuvent être modifiés, mais elle sera contrainte de toujours passer par un même point (le centre de la rotule). Ainsi, en plus des mouvements d'enfoncement/retrait et les changements d'orientation, les mouvements de rotation de la tige autour de son axe peuvent également être effectués.

Ces mouvements de translation selon l'axe de la tige et de rotation autour de l'axe de la tige sont ensuite transmis fidèlement au dispositif haptique puisque la première extrémité de la tige est montée fixe sur l'extrémité libre du dispositif haptique. Le dispositif haptique est alors capable de les mesurer.

Bien entendu, lorsqu'il est fait référence à l'instrument chirurgical, il s'agit des différents instruments chirurgicaux qui peuvent être utilisés lors de la chirurgie vitréo-rétinienne.

Selon différentes caractéristiques de l'invention qui pourront être prises ensemble ou séparément :
- l'interface de pilotage comprend ledit organe de préhension, l'organe de préhension et la tige comprenant respectivement des moyens de détection aptes à détecter une pression exercée par un utilisateur sur ledit organe de préhension,
- un premier moyen de détection est un aimant et un deuxième moyen de détection est un capteur à effet Hall,
- l'organe de préhension comprend un préhenseur déformable et une partie mobile sur laquelle est monté fixe ledit aimant, ladite partie mobile étant apte à se déplacer lorsque le préhenseur se déforme,
- la tige se présente sous la forme d'un corps creux délimitant intérieurement un logement,
- ledit deuxième moyen de détection est disposé dans le logement,
- la surface fixe comprend une ouverture et le moyen de guidage comprend un organe sphérique et un élément d'adaptation de l'organe sphérique dans l'ouverture, ladite ouverture, l'élément d'adaptation et ledit organe sphérique formant la liaison rotule,
- la tige est de forme cylindrique, l'organe sphérique étant creux et présentant un pallier lisse épousant la forme de la tige de manière à former la liaison glissière,
- la tige comprend des moyens d'alimentation du deuxième moyen de détection disposés dans le logement, lesdits moyens d'alimentation étant électriquement connectés au deuxième moyen de détection et aptes à recevoir un signal électrique émis par le deuxième moyen de détection lorsqu'une pression est exercée par un utilisateur sur ledit organe de préhension,
- le dispositif de guidage comprend un collecteur tournant disposé de manière coaxiale autour de la tige, ledit collecteur comprenant une bague intérieure fixée sur la tige et une bague extérieure fixée sur un axe de la chaîne articulée sur lequel se trouve l'extrémité libre du dispositif haptique,
- l'interface de pilotage comprend une housse stérile interposée entre l'organe de préhension et la deuxième extrémité de la tige.

L'invention concerne en outre un système robotisé pour la chirurgie vitréo-rétinienne comprenant :
- une plateforme robotisée comprenant au moins un bras robotisé destiné à porter au moins un instrument chirurgical, ledit bras robotisé comportant un module d'actionnement comprenant au moins un actionneur,
- une console comprenant une interface de pilotage telle que précédemment décrite,
- un module de traitement connecté à l'interface de pilotage et au module d'actionnement par un réseau de communication, ledit module de traitement comprenant au moins un processeur, une mémoire et un logiciel pour analyser les mesures effectuées par le dispositif haptique et calculer et donner des consignes de mouvements au bras robotisé,
ladite plateforme étant configurée de sorte que les mouvements appliqués à la tige sont reproduits sur ledit instrument chirurgical au moyen du bras robotisé.

De préférence, le système robotisé est configuré de sorte que les pressions exercées sur l'organe de préhension et les mouvements qui lui sont appliqués sont reproduits par l'instrument chirurgical au moyen du bras robotisé.

### Brève description des figures

D'autres objets, caractéristiques et avantages de l'invention apparaîtront plus clairement dans la description qui suit, faite en référence aux figures annexées, dans lesquelles :
- la figure 1 illustre une vue en perspective d'une interface de pilotage selon l'invention ;
- la figure 2 illustre une vue en coupe, en perspective, d'un dispositif de guidage de l'interface de pilotage selon l'invention ;
- la figure 3 est une représentation schématique de l'interface de pilotage selon l'invention illustrant les différentes liaisons cinématiques interconnectant les pièces les unes aux autres ;
- la figure 4 illustre une vue en perspective d'un organe de préhension adapté à être utilisé dans l'interface de pilotage selon l'invention ;
- la figure 5a illustre un chirurgien en train de manipuler l'organe de préhension ;
- la figure 5b est une représentation schématique de l'interface de pilotage selon l'invention, notamment muni d'un mécanisme de sous-main ;
- la figure 6 est une représentation schématique de l'interface de pilotage selon l'invention illustrant les liaisons mécaniques dans le dispositif de guidage ;
- la figure 7 est une représentation schématique du calcul et de la transmission de données entre l'interface de pilotage et la plateforme robotisée.

### Description détaillée de l'invention

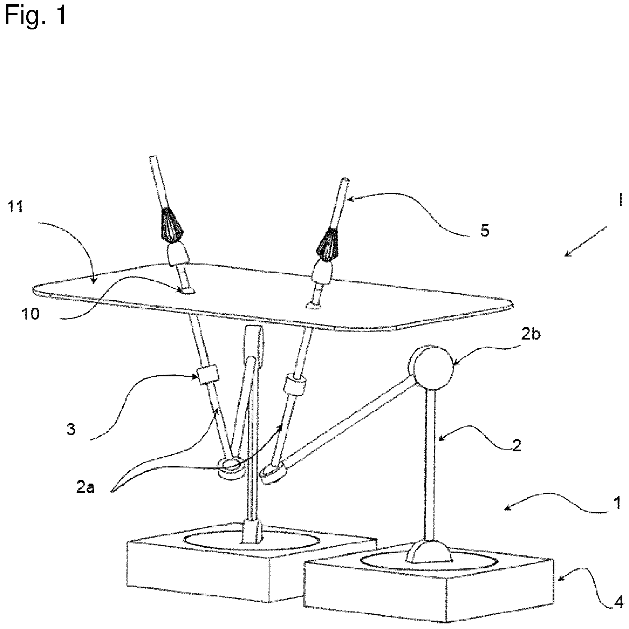

En référence à la figure 1, l'invention concerne une interface de pilotage I d'un système robotisé SR pour la chirurgie vitréo-rétinienne.

Le système robotisé SR dont il s'agit est destiné à être utilisé par un praticien, notamment par un chirurgien dans le cadre d'une opération de chirurgie vitréo-rétinienne (seules ses interfaces de pilotages sont illustrées dans l'exemple de réalisation de la figure 1). Il comprend une plateforme robotisée PR comportant au moins un bras robotisé BR servant à manipuler un instrument chirurgical. De préférence, la plateforme robotisée PR comprend deux bras robotisés BR ce qui permet de manipuler simultanément au moins deux instruments chirurgicaux. En outre, chacun des bras robotisés BR peut comprendre un module d'actionnement dudit bras robotisé BR. Ce module d'actionnement comprend un nombre d'actionneurs adapté à la commande du bras robotisé.

En outre, le système robotisé SR comprend au moins une interface de pilotage I associée au bras robotisé BR, étant entendu que si la plateforme robotisée PR comprend deux bras robotisés, le système robotisé SR comprend au moins une interface de pilotage, de préférence deux interfaces de pilotage I, chaque interface de pilotage I étant associée à un bras robotisé BR. Dans l'exemple de réalisation illustré sur la figure 1, le système robotisé SR comprend deux interfaces de pilotages I.

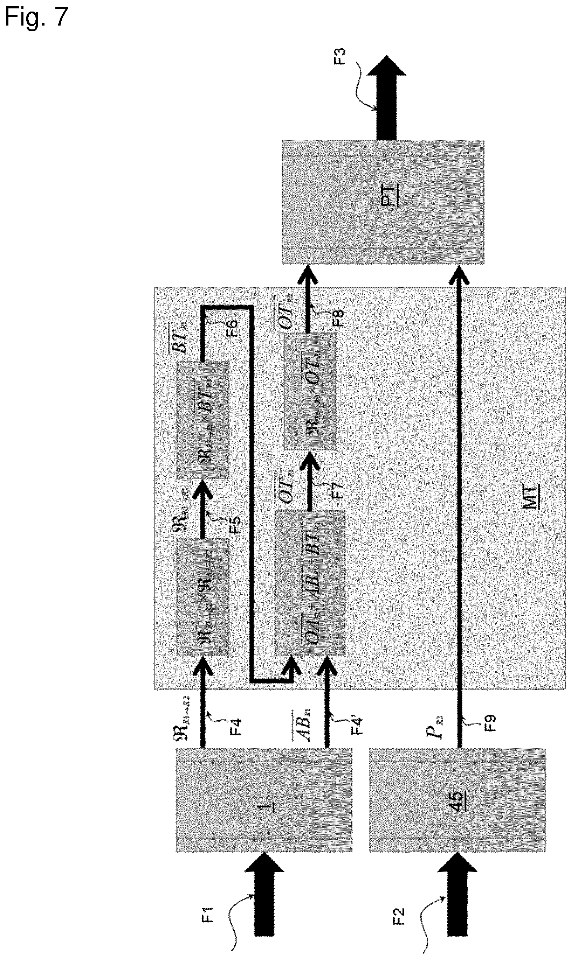

En outre, le système robotisé SR comprend un module de traitement MT apte à recevoir et traiter les données de mesure reçues par l'(les)interfaces de pilotage I de manière à générer un mouvement de l'instrument chirurgical fonction des mouvements opérés avec l'(es)interface(s) de pilotage. Ce module de traitement sera décrit plus en détail dans la description relative à la figure 7.

L'instrument chirurgical est tout instrument chirurgical susceptible d'être utilisé lors de la chirurgie vitréo-rétinienne. Il va sans dire que l'instrument est en cela apte à passer à travers un trocart. À titre de rappel, le trocart se présente sous la forme d'une tige cylindrique creuse et pointue qui s'étend selon un axe longitudinal. L'instrument est non seulement apte à passer à travers le trocart, mais également apte, au choix ou en combinaison, à couper, cautériser, injecter, aspirer, etc. À cet égard, le praticien est généralement amené à effectuer un pincement pour actionner l'instrument, c'est-à-dire qu'il effectue le geste de pincement pour que l'instrument coupe, cautérise, injecte, aspire, etc. Dans la suite, les mouvements de l'instrument chirurgical sont décrits par rapport à l'axe longitudinal du trocart.

L'interface de pilotage I comprend un dispositif haptique 1, un dispositif de guidage 10 et avantageusement un organe 5 de préhension monté de manière amovible sur le dispositif de guidage.

Le dispositif haptique 1 est un dispositif à retour de force permettant d'effectuer des mesures de positionnement dans l'espace de très grande précision. En d'autres termes, le dispositif haptique 1 a pour but de mesurer les positions avec une très grande fidélité.

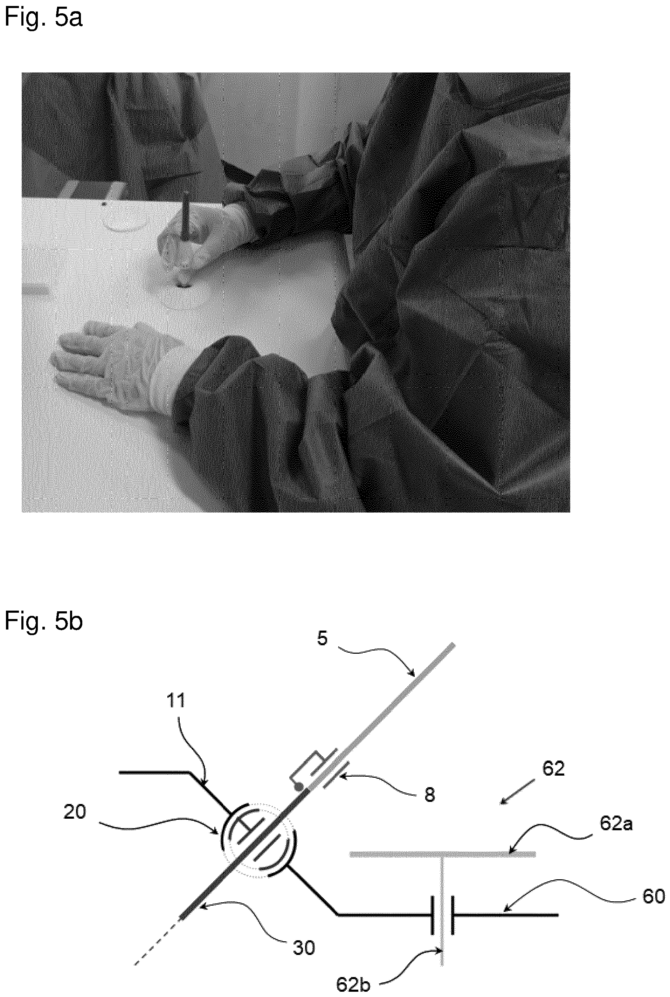

À cet égard, il comprend une chaîne articulée 2 comportant une pluralité de bras articulés 2a reliés les uns aux autres par des articulations 2b conçues de manière à autoriser la chaîne articulée 2 à effectuer des mouvements dans tous les degrés de liberté. La chaîne articulée comprend au moins deux bras articulés 2a. Chaque articulation 2b est munie de son propre moteur ce qui permet de créer des efforts. Chaque articulation 2b comprend son propre capteur de position angulaire ce qui permet de mesurer sa position. Une extrémité de la chaîne articulée 2 est reliée à un support 4, qui est de préférence fixe par rapport à une console 60 qui sera décrite plus en détail dans la description relative à la figure 5b. Une extrémité libre 3 de la chaîne articulée est, comme cela sera vu plus en détail dans la suite, fixée au dispositif de guidage 10. Ainsi, le dispositif haptique 1 comprend tous les éléments compris entre l'extrémité libre 3 et le support 4, y compris l'extrémité libre 3 et le support 4.

Le dispositif de guidage 10 comprend une surface fixe 11, une tige 30 et un moyen de guidage 20 par l'intermédiaire duquel coopèrent la surface 11 et la tige 30.

La surface fixe 11 permet d'assurer le maintien du moyen de guidage 20, c'est-à-dire qu'elle en assure le support mécanique. Elle est fixe par rapport au support 4 du dispositif haptique 1. Autrement dit, il n'existe pas de mouvement du dispositif de guidage 10 par rapport au dispositif haptique 1. Elle est intégrée à la console 60 mentionnée précédemment et est fixe par rapport à ladite console 60.

Dans l'exemple de réalisation illustré à la figure 1, la surface fixe 11 est plane. Cependant, ceci n'est nullement limitatif puisque la surface fixe 11 pourrait aussi être de toute autre forme, par exemple, courbe. Ce qui importe ici est que la surface fixe 11 assure le maintien du moyen de guidage 20 tout en étant fixe par rapport au support 4 du dispositif haptique 1.

Comme cela a été mentionné précédemment, la tige 30 coopère avec la surface fixe 11 par l'intermédiaire du moyen de guidage 20.

À cet égard, le moyen de guidage 20 est monté sur la surface fixe 11 par une liaison rotule 21 tandis que la tige 30 est montée sur ledit moyen de guidage 20 par une liaison glissière 25 d'axe correspondant à celui de la tige 30. En étant ainsi agencés les uns par rapport aux autres, la tige 30, le moyen de guidage 20 et la surface 11 forment une liaison rotule glissante qui sera décrite plus en détail dans la suite.

Incidemment, la tige 30 du dispositif de guidage peut être manipulée selon un mouvement de changement d'orientation autour de la liaison rotule 21, selon un mouvement de rotation autour de l'axe de la tige et selon un mouvement de translation d'axe correspondant à l'axe de la tige. Dans ce contexte, la tige 30 est du point de vue du chirurgien assimilable à la tige d'un instrument chirurgical utilisé lors de la chirurgie vitréo-rétinienne. Le mouvement de rotation de la tige 30 autour de son axe, c'est-à-dire le mouvement de rotation de la tige 30 sur elle-même, correspond à un mouvement de rotation de l'instrument sur lui-même. Le mouvement de changement d'orientation de la tige 30 correspond à un changement d'orientation de l'instrument et de l'axe du trocart dans l'œil du patient, le trocart suivant nécessairement les changements d'orientation de l'instrument chirurgical. En effet, le trocart est en liaison rotule avec l'œil. Enfin, le mouvement de translation de la tige 30 le long de son axe correspond à un enfoncement/retrait de l'instrument dans/du trocart, l'enfoncement ou le retrait dépendant du sens dans lequel le mouvement de translation est opéré. Cela étant dit, quel que soit le mouvement opéré, la tige 30 est contrainte de toujours passer par un centre de la liaison rotule.

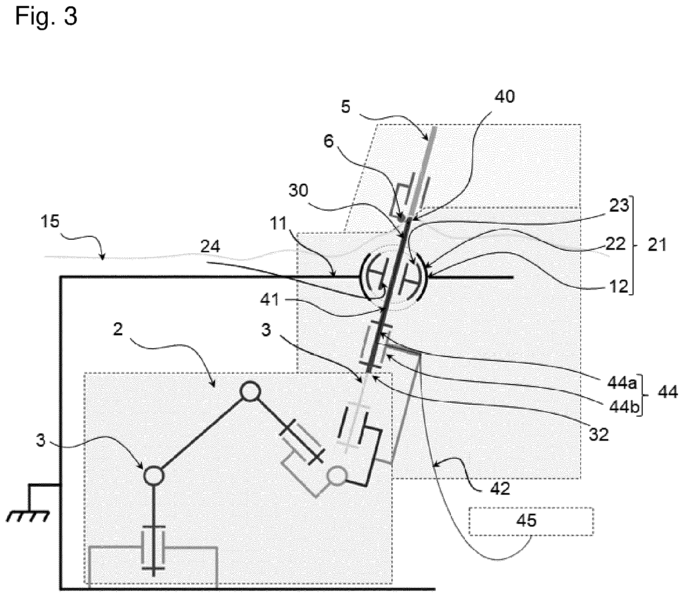

En outre, comme cela peut être mieux vu à la figure 3, la tige 30 comprend une première extrémité 32 montée fixe sur l'extrémité libre 3 de la chaîne articulée du dispositif haptique. C'est donc par la première extrémité 32 de la tige que le dispositif de guidage 10 est relié au dispositif haptique 1. Comme la première extrémité 32 de la tige est montée fixe sur l'extrémité libre 3 du dispositif haptique 1, les articulations 3 de la chaîne articulée 2 s'adaptent aux mouvements qui sont effectués par la tige 30. Ces mouvements sont mesurés de manière précise et les données issues de ces mesures sont transmises au module de traitement MT.

En manipulant la tige 30, le praticien effectue les mêmes mouvements qu'il aurait faits s'il avait directement manipulé l'instrument tout en bénéficiant de l'assistance apportée par le système robotisé SR.

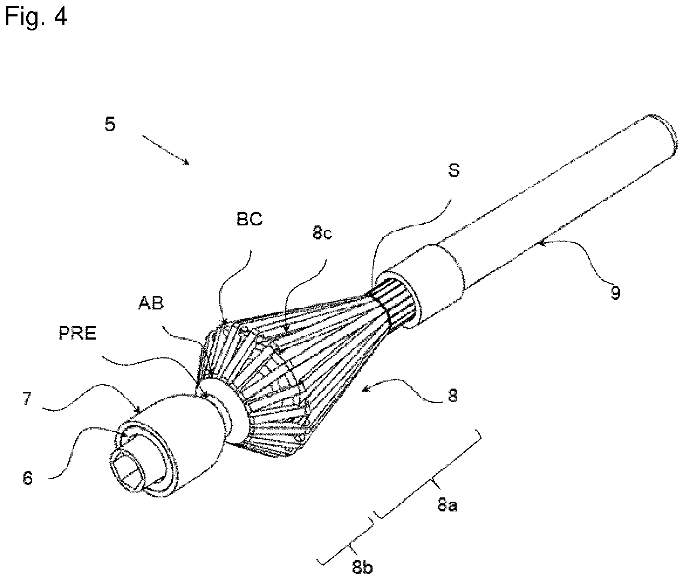

Cela étant, en pratique, le praticien peut avantageusement contrôler les mouvements de la tige 30 au moyen d'un organe 5 de préhension. L'organe 5 de préhension, qui sera décrit plus en détail en relation avec la description relative à la figure 4, se présente sous une forme similaire à celle de l'instrument chirurgical, ce qui rend fidèle l'expérience du praticien. En plus de reprendre la même forme qu'un instrument chirurgical classique, l'organe 5 de préhension est également préférablement stérile. Le terme « stérile » s'entend ici au sens médical et implique donc que l'organe 5 de préhension a fait l'objet d'un procédé préalable de stérilisation et est de ce fait exempt de germes.

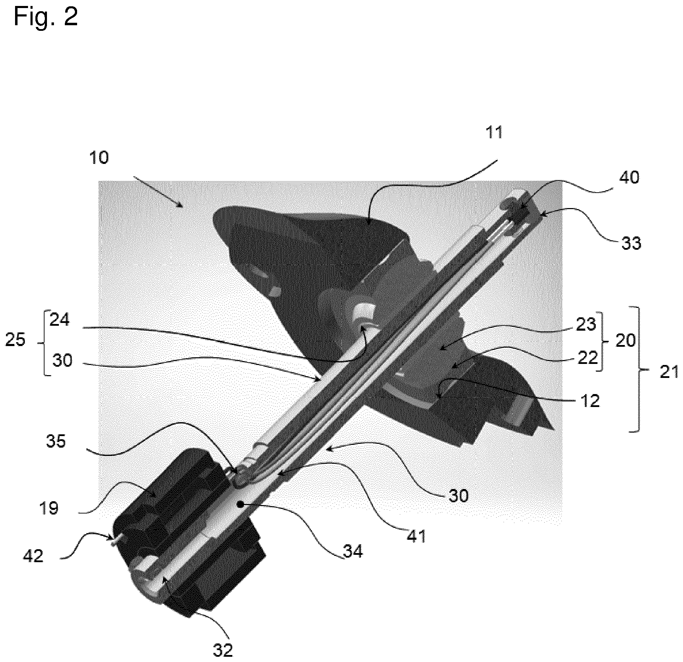

Comme cela est illustré dans l'exemple de réalisation de la figure 2, la tige 30 comprend une deuxième extrémité 33 sur laquelle est destiné à être monté ledit organe 5 de préhension. La tige 30 est ainsi liée à l'organe 5 de préhension de manière rigide, ce qui permet, lorsque l'organe 5 de préhension est présent, de transmettre fidèlement les mouvements dudit organe 5 de préhension à ladite tige 30 tout en permettant au chirurgien d'avoir des sensations similaires à celles qu'il aurait en manipulant l'instrument chirurgical.

En référence à la figure 2, l'agencement de la tige 30 au sein du dispositif de guidage 10 et le fonctionnement du dispositif de guidage 10 sont plus précisément décrits.

La surface fixe 11 comprend une ouverture 12 et le moyen de guidage 20 comprend un organe sphérique 23 qui coopère avec l'ouverture 12 par l'intermédiaire d'un élément d'adaptation 22 pour former la liaison rotule 21. L'élément d'adaptation 22 est interposé entre l'ouverture 12 et l'organe sphérique 23. Il consiste en une pièce mécanique comprenant une bague extérieure de forme cylindrique et un logement intérieur de forme sphérique dans lequel évolue librement l'organe sphérique 23. Les dimensions de l'élément d'adaptation 22 sont choisies de sorte que ledit élément d'adaptation épouse extérieurement la forme de l'ouverture 12 et intérieurement la forme de l'organe sphérique 23. Cette configuration permet, notamment du fait que l'organe sphérique 23 peut évoluer librement dans le logement intérieur de l'élément d'adaptation 22, de faire varier l'orientation de la tige 30. En outre, il résulte de cette configuration que le centre de la liaison rotule 21 est le centre de l'organe sphérique 23.

La configuration précitée n'est nullement limitative et l'homme du métier peut envisager d'autres configurations pour concevoir la liaison rotule 21.

Préférentiellement, l'organe sphérique 23 est une sphère en acier chromé.

Dans l'exemple de réalisation illustré à la figure 2, la tige 30 est de forme cylindrique tandis que l'organe sphérique 23 est creux et présente une pièce intérieure 24 épousant la forme de la tige 30 de manière à former la liaison glissière 25. Par exemple, la pièce intérieure 24 peut consister en un palier lisse réalisé en iglidur ^{®}. La pièce intérieure 24 présente donc des contours de forme cylindrique dont les dimensions sont choisies de sorte qu'elle soit le plus ajustée que possible autour de la tige 30 sans toutefois empêcher à ladite tige 30 de glisser. Là encore, la configuration illustrée n'est nullement limitative. On pourrait également prévoir une tige 30 se présentant sous la forme d'un pavé droit, la surface intérieure 24 présenterait alors des contours sous la forme d'un pavé creux. Il existe de très nombreuses autres façons de réaliser la liaison glissière 25 entre l'organe sphérique 23 et la tige 30 qui sont à la portée de l'homme de l'art sans toutefois sortir du concept inventif de l'invention.

En combinant la liaison rotule 21 et la liaison glissière 25, on forme ainsi une liaison rotule glissante qui permet au praticien de mimer à la fois des mouvements de retrait/enfoncement de l'instrument chirurgical à travers le trocart, les changements d'orientation de l'instrument chirurgical et de l'axe du trocart, qui pour rappel suit les mouvements de changements d'orientation de l'instrument chirurgical dans l'œil, et les mouvements de rotation de l'instrument chirurgical autour de cet axe.

Comme illustré encore sur la figure 2, la tige 30 se présente avantageusement sous la forme d'un corps creux délimitant intérieurement un logement 34. Autrement dit, la tige 30 elle-même se présente donc dans l'ensemble sous la forme d'un cylindre creux. Le logement 34 ainsi formé permet avantageusement d'accueillir un deuxième moyen 40 de détection fonctionnant en synergie avec un premier moyen de détection 6 compris dans l'organe 5 de préhension. Le logement 34 permet également de recevoir des moyens d'alimentation 41 du deuxième moyen de détection 40. L'intérêt desdits premier et deuxième moyens 6, 40 de détection et des moyens d'alimentation 41 est décrit ci-après dans la description relative à la figure 4.

Les moyens d'alimentation 41 du deuxième moyen 40 de détection sont de préférence des fils électriques. Afin de prévenir tout entortillement des fils, c'est-à-dire afin d'empêcher que les fils ne s'emmêlent, le dispositif de guidage 10 comprend avantageusement un collecteur tournant 44 disposé de manière coaxiale autour de la tige 30. Le collecteur tournant 44 permet de connecter électriquement les fils 41 à une carte électronique 45 ayant pour rôle de traiter les données issues du deuxième moyen 40 de détection. Plus précisément, il permet de connecter électriquement les fils 41 situés dans la tige, qui du fait de la liaison rotule glissante est tournante, à d'autres fils 42 reliés à la carte électronique 45, qui, elle, demeure fixe par rapport à la surface 11, contrairement à la tige 30. À ce propos, précisons que la tige 30 comprend un orifice 35 pour le passage des fils 41 depuis le logement 34 vers le collecteur 44.

Avantageusement, le praticien peut effectuer de multiples rotations de la tige 30, pour ainsi dire des rotations infinies, sans que les fils 41 ne s'emmêlent. À cet égard, le collecteur 44 est lui-même une liaison pivot. Comme représenté schématiquement sur la figure 3, le collecteur 44 comprend une bague intérieure 44a fixée sur la tige 30 et une bague extérieure 44b fixée sur un axe du bras articulé 2a sur lequel se trouve l'extrémité libre 3 du dispositif haptique. Des moyens pour empêcher la translation du collecteur 44 par rapport à la tige 30 sont également prévus au niveau de la tige. De tels moyens peuvent par exemple consister en des protubérances disposées sur une surface extérieure de la tige de chaque côté du collecteur 44. Cela permet d'éviter que les fils 41 ne s'étirent et subséquemment se détériorent.

En référence aux figures et en particulier aux figures 1 et 4, l'interface de pilotage I comprend très avantageusement l'organe 5 de préhension. L'utilisation de l'organe 5 de préhension présente de nombreux avantages qui seront mieux compris dans la suite.

L'organe 5 de préhension n'est pas monté à demeure sur le dispositif de guidage 10. Celui-ci est en effet monté de manière amovible sur le dispositif de guidage 10. Le fabricant peut donc tout à fait commercialiser l'interface de pilotage I sans l'organe 5 de préhension.

Comme illustré dans l'exemple de la figure 4, l'organe 5 de préhension se présente sous la forme d'un stylet similaire, en termes de forme et de dimensions uniquement, à un instrument standard pour la chirurgie vitréo-rétinienne. L'organe 5 de préhension n'est pas un instrument chirurgical et n'est pas dévolu à être utilisé directement sur le patient. Il s'agit d'un instrument de pilotage, c'est-à-dire ayant une fonction de pilotage, notamment de pilotage du pincement. Il fonctionne en coopération avec le reste de l'interface de pilotage I aux fins de la détection et de la mesure du pincement.

L'organe 5 de préhension comprend une portion allongée 9, un préhenseur 8 déformable et une partie mobile 7 situés côte à côte dans cet ordre.

La portion allongée 9, de forme sensiblement cylindrique, coopère avec le préhenseur 8 déformable pour fournir au praticien des sensations en terme de toucher qui sont équivalentes à celles qu'il aurait en manipulant un instrument chirurgical. Sur la figure 4, seule une enveloppe extérieure de la portion allongée 9 est visible mais celle-ci comprend une âme non visible qui s'étend bien au-delà de l'enveloppe extérieure et plus précisément au moins jusqu'à la partie mobile 7, comme cela sera vu dans la suite.

Le préhenseur 8 déformable présente une forme de diamant, c'est-à-dire une forme bipyramidale, dans laquelle les pyramides ont une base commune BC et dans laquelle l'une des pyramides est tronquée. Le préhenseur 8 est donc formé d'une pyramide complète 8a et d'une pyramide tronquée 8b. Le sommet S de la pyramide complète est situé du côté de la portion allongée 9 tandis que la base de plus faible aire, ci-après intitulée « autre base » AB, de la pyramide tronquée est située à proximité de la partie mobile 7. Chacune des pyramides est formée par une pluralité de languettes 8c qui confèrent au préhenseur 8 son caractère déformable.

En outre, il convient de préciser que si l'autre base AB de la pyramide tronquée 8b est reliée de manière rigide à la partie mobile 7 par l'intermédiaire d'une portion rétrécie PRE, l'autre base AB et la partie mobile 7 sont mobiles par rapport à la portion allongée 9, notamment son âme (non visible). En effet, l'autre base AB et la partie mobile 7 sont toutes les deux en liaison glissière avec l'âme de la portion allongée 9 qui, même si cela ne peut être distingué sur la figure 4, s'étend au moins jusqu'à la partie mobile 7. Cette liaison glissière, représentée de manière schématique à la figure 3, est formée par une ouverture pratiquée dans l'autre base AB et la partie mobile 7, leurs ouvertures respectives étant ajustées à la surface extérieure de l'âme de la portion allongée 9, c'est-à-dire qu'elles présentent des dimensions appropriées pour former la liaison glissière avec l'âme de la portion allongée 9.

Dans une telle configuration, lorsqu'une pression est exercée au niveau de la base commune BC, les languettes 8c étant plus fines au niveau de la base BC comparativement à leur épaisseur au niveau du sommet S et de l'autre base AB, le préhenseur 8 se déforme, ce qui permet à l'autre base AB et la partie mobile 7 de glisser simultanément le long de la portion allongée 9. À ce propos, l'allongement du préhenseur 8 peut être modifié en fonction de la pression exercée et du lieu où s'exerce cette pression sur ledit préhenseur 8. À pression constante, plus l'appui est exercé à proximité de la base commune BC plus le préhenseur 8 se déforme et donc s'allonge. Concomitamment, pour une position d'appui identique entre deux manipulations, plus la pression exercée, c'est-à-dire le taux d'appui est élevé, plus le préhenseur 8 se déforme et donc s'allonge.

La position d'appui, i.e. le lieu où est exercée la pression sur le préhenseur 8, et la pression exercée, i.e. le taux d'appui, choisis influencent donc le déplacement de la partie mobile 7 et en conséquent, comme cela sera vu dans la suite, le niveau de pincement souhaité. Ainsi, lorsque le praticien appui sur le préhenseur 8, il peut manipuler l'organe 5 de préhension comme il manipulerait l'instrument chirurgical lorsqu'il veut effectuer un pincement et adapte la position de ses doigts par rapport à la base commune BC en fonction du niveau de pincement qu'il veut obtenir. La figure 5a illustre une telle situation. La configuration précitée n'est qu'un exemple de mise en œuvre de l'organe 5 de préhension. L'homme du métier peut envisager toute autre solution qui permet de générer un déplacement de la partie mobile 7 lorsqu'une pression est exercée sur le préhenseur 8.

Ceci étant dit, si la position de préhension et le taux d'appui, déterminent le niveau de pincement souhaité, la manière dont l'allongement est détecté puis mesuré est explicitée dans ce qui suit.

De manière très avantageuse, l'organe 5 de préhension comprend un moyen 6 de détection, appelé dans la suite premier moyen 6 de détection, permettant de détecter les déplacements de la partie mobile 7. À cette fin, ledit premier moyen 6 de détection est monté fixe sur la partie mobile 7 de sorte que tout déplacement de la partie mobile 7 le long de l'âme de la portion allongée 9 induise automatiquement un déplacement du premier moyen 6 de détection. Précisons également qu'une fois l'organe 5 de préhension monté sur la tige 30, la partie mobile 7 devient aussi mobile par rapport à la tige 30 et donc par rapport au deuxième moyen 40 de détection. Ceci est d'importance dans la mesure où ce sont les mouvements du premier moyen 6 de détection qui peuvent être détectés par le deuxième moyen 40 de détection du dispositif de guidage 10 et subséquemment être mesurés par celui-ci. Ainsi, dès qu'une pression suffisante est exercée sur le préhenseur 8 pour que la partie mobile 7 et donc le premier moyen 6 de détection se déplacent, le déplacement du premier moyen 6 de détection peut être mesuré par le deuxième moyen 40 de détection. Les premier et deuxième 6, 40 moyens de détection opèrent donc en synergie pour détecter un appui exercé sur l'organe 5 de préhension. C'est ce qui confère à l'organe 5 de préhension sa fonction de pilotage, notamment de pilotage du pincement effectué par le praticien.

De manière préférentielle, le premier moyen 6 de détection est un aimant et le deuxième moyen 40 de détection est un capteur à effet Hall. Plus précisément l'aimant est un aimant permanent. Ainsi, lorsqu'une pression suffisante est exercée sur le préhenseur 8 pour que l'aimant 6 se déplace, le champ magnétique de l'aimant se déplace et varie donc aux yeux du capteur à effet Hall 40, qui pour rappel est situé dans la tige 30. Le taux d'appui peut ainsi être déduit du signal issu du capteur à effet Hall.

L'avantage d'une telle configuration par rapport à un autre système de détection est que l'organe 5 de préhension est ainsi dépourvu d'électronique, toute la partie électronique étant dans la tige 30 et donc dans le dispositif de guidage 10. Il est donc possible de détecter les appuis effectués sur l'organe 5 de préhension, quand bien même celui-ci est dépourvu d'électronique. De plus, le coût de l'organe 5 de préhension peut rester faible et celui-ci peut donc plus facilement être utilisé comme un consommable. Ainsi, on s'assure que les conditions de stérilisation requises pour l'organe 5 de préhension sont respectées à chaque intervention. De plus, comme l'organe 5 préhension est un consommable dépourvu d'électronique, il est plus facile à recycler.

De préférence, le capteur à effet Hall 40 est situé au niveau de ladite deuxième extrémité 33 dans le logement 34 de la tige. Ainsi, il se trouve au plus près de l'aimant 6, ce qui renforce sa capacité à détecter les mouvements dudit aimant même s'ils sont de faible amplitude. Bien entendu, le capteur à effet Hall 40, de même que l'aimant 6, peuvent être agencés de toute autre manière dans la mesure où une variation de la pression exercée sur l'organe 5 de préhension peut être détectée. De manière avantageuse, le logement 34 de la tige permet d'accueillir d'autres types de capteurs selon les fonctionnalités supplémentaires que l'on souhaite conférer à l'interface de pilotage I selon l'invention.

D'ailleurs, si la combinaison d'un aimant 6 avec un capteur à effet Hall 40 présente les avantages susmentionnés, d'autres solutions peuvent être envisagées pour détecter et mesurer les appuis du praticien sur l'organe 5 de préhension. On pourra avoir recours à un capteur électrique passif inductif de déplacement linéaire (*Linear Variable Differential Transformer* en anglais). Ce type de capteurs comprend un transformateur cylindrique et un noyau et présente une réponse proportionnelle au déplacement du noyau dans le transformateur. On pourra, par exemple, avoir recours à une jauge de déformation. La jauge de déformation permet en effet de traduire la déformation d'une pièce par une variation de résistance électrique. Cela étant dit, l'homme du métier pourra avoir recours à tout capteur qui permet d'avoir un retour de position.

Le signal électrique SE émis par le capteur à effet Hall 40 est transmis à la carte électronique 45 par les fils 41, le collecteur 44 et les fils 42, pour que celle-ci les traite. Les données du capteur pourraient être transmises à la carte électronique 45 par tout autre moyen connu de l'homme de l'art. Les données traitées par la carte électronique 45 sont ensuite transmises au module de traitement MT qui les traite et envoie des consignes spécifiques à la plateforme robotisée PR de sorte que la pression exercée par le praticien sur l'organe 5 de préhension puisse être reproduite sur l'instrument chirurgical au moyen du bras robotisé BR. Le praticien n'a donc pas besoin d'apprendre de nouveaux gestes pour que l'instrument chirurgical effectue un pincement puisqu'il manipule l'organe 5 de préhension de la même façon qu'il aurait manipulé l'instrument chirurgical lui-même.

Comme illustré à la figure 5b, l'interface de pilotage 1 peut comprendre une console 60. La console 60 consiste en un pupitre, ajustable en hauteur, dans lequel sont intégrés la surface fixe 11, le moyen de guidage 10 et la tige 30 selon la configuration précédemment décrite. C'est au niveau de la surface 11 que la console 60 est reliée au reste de l'interface de pilotage I. À ce propos, la surface fixe 11 peut être intégrale avec la console 60 ou être une pièce rapportée qui est préalablement fixée sur la console 60. Lorsque le chirurgien est installé au niveau de l'interface de pilotage I, comme cela est illustré à la figure 5a, la console 60 lui permet d'avoir les appuis et la stabilité nécessaires pour pouvoir manipuler la tige 30, et le cas échéant l'organe de préhension 5. La console 60 constitue une limite physique entre le chirurgien et le dispositif haptique 1 puisque le dispositif haptique 1 est situé sous la console 60.

De préférence, la console 60 dispose de reposes poignet (non illustrés) sur lesquels le praticien peut s'appuyer pour être stable. De plus, la console 60 peut également comprendre un mécanisme de sous-main 62 ajustable en hauteur. Ce mécanisme de sous-main 62 peut être placé sous l'organe 5 de préhension afin de faciliter la préhension de ce dernier tout en étant adaptable à la morphologie du praticien. Le mécanisme de sous-main 62 comprend au moins une surface d'appui 62a et un tronc 62b s'étendant depuis la console 60. La hauteur du mécanisme de sous-main 62 peut être ajustée en réalisant une liaison glissière entre le tronc 62b et la console 60. De préférence la liaison glissière est motorisée ce qui permet d'ajuster la hauteur du mécanisme de sous-main 62 en fonction du type d'instrument utilisé et de la chirurgie réalisée.

Précisons encore, en référence à la figure 3, que l'interface de pilotage I peut en outre comprendre une housse 15 stérile. La housse 15 stérile peut être interposée entre l'organe 5 de préhension et le reste des éléments de l'interface de pilotage I. Cela permet de conserver un environnement stérile depuis la deuxième extrémité 33 de la tige, où est monté l'organe 5 de préhension, jusqu'au patient. La housse 15 constitue, à ce titre, une contrainte qui doit être prise en compte lors de la conception de l'interface de pilotage I. La housse 15 stérile comprend, de préférence, des éléments d'étanchéité (non illustrés) entre les deux parties.

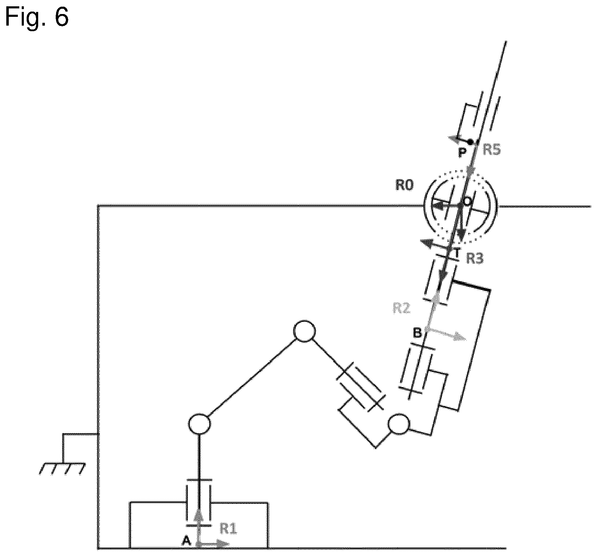

En référence à la figure 6, la cinématique et la géométrie du dispositif de guidage 10 sont décrites. Un repère R0 ayant pour origine O est associé au moyen de guidage 20. Un repère R1 ayant pour origine A est associé au support 4 du dispositif haptique, qui pour rappel est préférentiellement fixe par rapport à la console 60. Un repère R2 ayant pour origine B est associé à l'extrémité libre 3 de la chaîne articulée du dispositif haptique. Un repère R3 ayant pour origine T est associé à la tige 30. Un repère R5 ayant pour origine P est associé à la portion allongée 9 de l'organe de préhension 5.

Le point T correspond à un point de la tige 30 et au bout de l'instrument chirurgical dans l'œil du patient. Le chirurgien doit donc, pour modifier la position du bout de l'instrument chirurgical, modifier la position du point T dans R0. Pour mesurer cette position, un algorithme est implémenté. Celui-ci tient compte des transformations géométriques entre les repères R0 et R1, R1 et R2, R1 et R3 et R2 et R3. Comme cela a été mentionné précédemment la surface fixe 11 est fixe par rapport au support 4 du dispositif haptique 1, la transformation entre les repères R0 et R1 est donc constante et connue par conception. De même, l'extrémité libre 3 de la chaîne articulée étant fixe par rapport à la première extrémité 32 de la tige, la transformation géométrique entre R2 et R3 qui en résulte est constante et connue par conception. La transformation géométrique entre les repères R1 et R2 est quant à elle mesurée grâce au dispositif haptique 1. Par calculs effectués par le module de traitement MT, il est ainsi possible de connaître la transformation géométrique entre les repères R0 et R3.

Le point P du repère R5 est lié à la partie mobile 7. La position du point P varie après qu'une pression a été exercée sur l'organe 5 de préhension et que le préhenseur 8 a été déformé par rapport à la position du point P lorsqu'aucune pression n'est exercée sur l'organe 5 de préhension. Comme cela a été vu précédemment, cela traduit le niveau de pincement que le praticien aurait exercé sur l'instrument chirurgical à proprement parlé.

La mesure des positions de ces deux points T et P permet de connaître toutes les actions que le praticien souhaite que l'instrument chirurgical, situé en bout de chaîne, effectue dans l'œil du patient. Ces actions peuvent être effectuées par la plateforme robotisée PR à l'identique ou de manière améliorée. Il est à noter que la mesure seule de la position du point T dans le repère R0 serait déjà suffisante pour manipuler un instrument pour lequel le pincement n'est pas nécessaire, c'est à dire un instrument pour lequel seuls l'enfoncement/le retrait et le changement d'orientation sont requis. Dès lors que l'on souhaite effectuer un pincement, l'organe 5 de préhension devient nécessaire pour pouvoir mesurer le taux d'appui du praticien et il est alors nécessaire d'effectuer la mesure de la position du point P dans le repère R5.

À ce propos, les options offertes par l'interface de pilotage I selon l'invention sont nombreuses. La capacité du dispositif haptique 1 à générer des forces permet de fournir un retour au praticien. En effet, le dispositif haptique 1 permet de :
- limiter l'amplitude du mouvement de la tige 30, éventuellement celui de l'organe 5 de préhension afin de contraindre l'instrument à rester dans certaines zones,
- limiter la vitesse de déplacement de la tige 30, éventuellement celle de l'organe de 5 de préhension afin d'améliorer la précision et la stabilité des mouvements,
- adapter la vitesse de la tige 30, éventuellement celle de l'organe 5 de préhension, à celle de la plateforme robotisée PR, pour des raisons matérielles et/ou de sécurité,
- fournir un retour haptique basé sur des efforts mesurés par des capteurs situés au niveau de l'instrument monté sur la plateforme robotisée.

À cet égard, la transmission des données au sein du système robotisé, tel qu'illustré à la figure 7, répond au procédé décrit dans la suite.

Avantageusement, et comme mentionné précédemment le système robotisé SR comprend un module de traitement MT connecté à l'interface de pilotage I et au module d'actionnement, tels que précédemment décrits, par un réseau de communication (non illustré). Le module de traitement est muni d'un processeur et d'une mémoire. Il peut être tout type de moyen de traitement électronique ou informatique, par exemple un ordinateur ou tout appareil équipé d'un processeur et d'une mémoire, dès lors qu'il permet de traiter les données reçues du dispositif haptique 1 et de la carte électronique 45.

Le processeur est configuré pour analyser et/ou traiter les données obtenues à partir du dispositif haptique 1 et de la carte électronique 45. À cet égard, un logiciel de traitement peut être installé sur le processeur afin de réaliser ce traitement de manière automatique et en temps réel. Le logiciel comprend des algorithmes de géométrie spatiale et cinématique qui permettent de traduire les configurations dans l'espace adoptées par la tige 30 et éventuellement l'organe 5 de préhension en consignes pour chacun des actionneurs du module d'actionnement. Ces actionneurs agissent sur la plateforme robotisée PR pour commander l'instrument chirurgical. Comme cela a été mentionné dans la description relative à la figure 7, le logiciel détermine la position du point T dans le repère R0 et, le cas échéant, la position du point P dans repère R5. Ces algorithmes permettent notamment de faire respecter à l'instrument son passage dans le trocart. Les algorithmes permettent avantageusement de simplifier la mise en œuvre des options supplémentaires, vues ci-avant, qui peuvent être utilisées par le praticien en améliorant le geste initial effectué par le praticien.

La mémoire permet de recevoir et stocker, même temporairement, les données transmises et traitées par le module de traitement MT.

La flèche F1 indique que le praticien place la tige 30, et le cas échéant l'organe 5 de préhension, dans une certaine position et orientation, la flèche F2 indique que le praticien pince l'organe 5 de préhension, lorsque l'interface de pilotage I comprend ce dernier, et la flèche F3 indique que l'instrument chirurgical est placé dans la même position et orientation que la tige 30, le cas échéant la même position, orientation et pincement que l'organe 5 de préhension. Entre les évènements F1, le cas échéant avec F2, et l'évènement F3, la transmission des données s'effectue comme suit :
1) Les données mesurées par le dispositif haptique 1 et la carte électronique 45 sont transmises au module de traitement MT (flèches F4, F4' et F9),
2) Le module de traitement MT traite ces données via les algorithmes du logiciel et traduit les configurations adoptées par la tige 30 et éventuellement l'organe 5 de préhension en consignes pour chacun des actionneurs du module d'actionnement (processus entre F4 et F8, entre F4' et F8 et le cas échéant F9),
3) Les consignes sont envoyées au module d'actionnement,
4) Le module d'actionnement commande la plateforme robotisée afin que l'instrument chirurgical reproduise les mouvements effectués par le chirurgien (flèche F3).

Dans ce qui suit, le processus entre F4 et F8 est plus précisément décrit. Entre F4 et F5, le module de traitement MT a reçu la rotation qui existe entre les repères R1 et R2 (donnée par le dispositif haptique 1). Le module de traitement MT l'inverse et la compose avec la rotation qui existe entre les repères R3 et R2 (constante et connue par conception) pour déduire la rotation qui existe entre les repères R3 et R1. Entre F5 et F6, le module de traitement MT utilise la rotation qui existe entre les repères R3 et R1 et l'expression du vecteur BT dans le repère R3 (constante et connue par conception) pour déduire l'expression du vecteur BT dans le repère R1. Entre F6 et F7, le module de traitement MT reçoit l'expression du vecteur AB dans le repère R1 (donnée par le dispositif haptique 1) et l'additionne à celle du vecteur OA (constante et connue par conception) et à celle du vecteur BT (déduite après F6). Il en déduit l'expression du vecteur OT dans le repère R1. Entre F7 et F8, le module de traitement utilise la rotation qui existe entre les repères R1 et R0 (constante et connue par conception) et l'expression du vecteur OT dans le repère R1 pour en déduire l'expression du vecteur OT dans le repère R0. C'est exactement la donnée dont il a besoin pour savoir quelle position et quelle orientation le chirurgien veut donner à l'instrument.

## Revendications

1. Interface de pilotage (I) d'une plateforme robotisée pour la chirurgie vitréo-rétinienne comprenant un dispositif haptique (1) muni d'une chaîne articulée (2) ayant une extrémité libre (3), **caractérisée en ce qu'**elle comprend un dispositif de guidage (10) comportant :
- une surface fixe (11),
- un moyen de guidage (20) monté sur la surface fixe par une liaison rotule (21),
- une tige (30) montée sur ledit moyen de guidage (20) par une liaison glissière (25) d'axe correspondant à celui de la tige (30), ladite tige comportant une première extrémité (32) montée fixe sur l'extrémité libre (3) du dispositif haptique (1) et une deuxième extrémité (33) sur laquelle est destiné à être monté un organe (5) de préhension.

2. Interface de pilotage (I) selon la revendication 1, **caractérisée en ce qu'**elle comprend en outre ledit organe (5) de préhension, l'organe (5) de préhension et la tige (30) comprenant respectivement des moyens (6, 40) de détection aptes à détecter une pression exercée par un utilisateur sur ledit organe de préhension.

3. Interface de pilotage (I) selon la revendication 2, dans laquelle un premier moyen (6) de détection est un aimant et un deuxième moyen de détection (40) est un capteur à effet Hall.

4. Interface de pilotage (I) selon la revendication 3, dans laquelle l'organe de préhension (5) comprend un préhenseur (8) déformable et une partie mobile (7) sur laquelle est monté fixe ledit aimant (6), ladite partie mobile (7) étant apte à se déplacer lorsque le préhenseur (8) se déforme.

5. Interface de pilotage (I) selon l'une des revendications 3 à 4, dans laquelle la tige (30) se présente sous la forme d'un corps creux délimitant intérieurement un logement (34), ledit deuxième moyen de détection (40) étant disposé dans le logement (34).

6. Interface de pilotage (I) selon l'une quelconque des revendications précédentes, dans laquelle la surface fixe (11) comprend une ouverture (12) et le moyen de guidage (20) comprend un organe sphérique (23) et un élément d'adaptation (22) de l'organe sphérique dans l'ouverture (12), ladite ouverture (12), l'élément d'adaptation (22) et ledit organe sphérique (23) formant la liaison rotule (21).

7. Interface de pilotage (I) selon la revendication 6, dans laquelle la tige (30) est de forme cylindrique, l'organe sphérique (23) étant creux et présentant un pallier lisse (24) épousant la forme de la tige (30) de manière à former la liaison glissière (25).

8. Interface de pilotage (I) selon l'une quelconque des revendications 5 à 7, dans laquelle la tige (30) comprend des moyens d'alimentation (41) du deuxième moyen de détection (40) disposés dans le logement (34), lesdits moyens d'alimentation (41) étant électriquement connectés au deuxième moyen de détection et aptes à recevoir un signal électrique (SE) émis par le deuxième moyen de détection (40) lorsqu'une pression est exercée par un utilisateur sur ledit organe (5) de préhension.

9. Interface de pilotage (I) selon la revendication précédente, dans laquelle le dispositif de guidage (10) comprend un collecteur tournant (44) disposé de manière coaxiale autour de la tige (30), ledit collecteur (44) comprenant une bague intérieure (44a) fixée sur la tige (30) et une bague extérieure (44b) fixée sur un axe de la chaîne articulée (2) sur lequel se trouve l'extrémité libre 3 du dispositif haptique(1).

10. Interface de pilotage (I) selon l'une quelconque des revendications 2 à 9, comprenant une housse (15) stérile interposée entre l'organe (5) de préhension et la deuxième extrémité (33) de la tige.

11. Système robotisé (SR) pour la chirurgie vitréo-rétinienne comprenant :
- une plateforme robotisée (PR) comprenant au moins un bras robotisé (BR) destiné à porter au moins un instrument chirurgical, ledit bras robotisé (BR) comportant un module d'actionnement comprenant au moins un actionneur,
- une console (60) comprenant une interface de pilotage (I) de la plateforme robotisée selon l'une quelconque des revendications précédentes,
- un module de traitement (MT) connecté à l'interface de pilotage (I) et au module d'actionnement par un réseau de communication, ledit module de traitement (MT) comprenant au moins un processeur (52), une mémoire (53) et un logiciel pour analyser les mesures effectuées par le dispositif haptique (1) et calculer et donner des consignes de mouvements au bras robotisé (BR),
ladite plateforme étant configurée de sorte que les mouvements appliqués à la tige (30) sont reproduits sur ledit instrument chirurgical au moyen du bras robotisé (BR).

12. Système robotisé (SR) selon la revendication 11 lorsqu'elle dépend des revendications 2 à 10, dans lequel système robotisé (SR) est configuré de sorte que les pressions exercées sur l'organe de préhension (5) et les mouvements qui lui sont appliqués sont reproduits par l'instrument chirurgical au moyen du bras robotisé (BR).

## Patentansprüche

1. Steuerschnittstelle (I) einer Roboterplattform für die vitreoretinale Chirurgie, umfassend eine haptische Vorrichtung (1), die mit einer Gelenkkette (2) versehen ist, die ein freies Ende (3) aufweist, **dadurch gekennzeichnet, dass** sie eine Führungsvorrichtung (10) umfasst, umfassend:
- eine feste Oberfläche (11),
- ein Führungsmittel (20), das durch eine Kugelgelenkverbindung (21) auf der festen Oberfläche montiert ist,
- einen Stift (30), der durch eine Gleitschienenverbindung (25) einer Achse, die jener des Stifts (30) entspricht, auf dem Führungsmittel (20) montiert ist, wobei der Stift ein erstes Ende (32) umfasst, das feststehend am freien Ende (3) der haptischen Vorrichtung (1) montiert ist, und ein zweites Ende (33), auf dem ein Greiforgan (5) dazu bestimmt ist, montiert zu werden.

2. Steuerschnittstelle (I) nach Anspruch 1, **dadurch gekennzeichnet, dass** sie weiter das Greiforgan (5) umfasst, wobei das Greiforgan (5) und der Stift (30) jeweils Erkennungsmittel (6, 40) umfassen, die imstande sind, einen Druck zu erkennen, der von einem Benutzer auf das Greiforgan ausgeübt wird.

3. Steuerschnittstelle (I) nach Anspruch 2, wobei ein erstes Erkennungsmittel (6) ein Magnet ist und ein zweites Erkennungsmittel (40) ein Hall-Effekt-Sensor ist.

4. Steuerschnittstelle (I) nach Anspruch 3, wobei das Greiforgan (5) einen verformbaren Greifer (8) und einen beweglichen Teil (7) umfasst, auf dem der Magnet (6) feststehend montiert ist, wobei der bewegliche Teil (7) zu verfahren imstande ist, wenn sich der Greifer (8) verformt.

5. Steuerschnittstelle (I) nach einem der Ansprüche 3 bis 4, wobei sich der Stift (30) in Form eines Hohlkörpers präsentiert, der im Inneren eine Aufnahme (34) begrenzt, wobei das zweite Erkennungsmittel (40) in der Aufnahme (34) angeordnet ist.

6. Steuerschnittstelle (I) nach einem der vorstehenden Ansprüche, wobei die feste Oberfläche (11) eine Öffnung (12) umfasst, und das Führungsmittel (20) ein sphärisches Organ (23) und ein Adapterelement (22) des sphärischen Organs in der Öffnung (12) umfasst, wobei die Öffnung (12), das Adapterelement (22) und das sphärische Organ (23) die Kugelgelenkverbindung (21) bilden.

7. Steuerschnittstelle (I) nach Anspruch 6, wobei der Stift (30) von zylindrischer Form ist, das sphärische Organ (23) hohl ist, und ein Gleitlager (24) aufweist, das sich an die Form des Stifts (30) anschmiegt, um das Gleitlager (25) zu bilden.

8. Steuerschnittstelle (I) nach einem der Ansprüche 5 bis 7, wobei der Stift (30) Versorgungsmittel (41) des zweiten Erkennungsmittels (40) umfasst, die in der Aufnahme (34) angeordnet sind, wobei die Versorgungsmittel (41) elektrisch mit dem zweiten Erkennungsmittel verbunden sind um imstande sind, um ein elektrisches Signal (SE) zu empfangen, das vom zweiten Erkennungsmittel (40) ausgegeben wird, wenn vom Benutzer ein Druck auf das Greiforgan (5) ausgeübt wird.

9. Steuerschnittstelle (I) nach dem vorstehenden Anspruch, wobei die Führungsvorrichtung (10) einen Schleifring (44) umfasst, der koaxial um den Stift (30) herum angeordnet ist, wobei der Schleifring (44) einen inneren Ring (44a) umfasst, der am Stift (30) befestigt ist, und einen äußeren Ring (44b), der auf einer Achse der Gelenkkette (2) befestigt ist, auf der sich das freie Ende 3 der haptischen Vorrichtung (1) befindet.

10. Steuerschnittstelle (I) nach einem der Ansprüche 2 bis 9, umfassend einen sterilen Überzug (15), der zwischen dem Greiforgan (5) und dem zweiten Ende (33) des Stifts eingesetzt ist.

11. Robotersystem (SR) für die vitreoretinale Chirurgie, umfassend:
- eine Roboterplattform (PR), die mindestens einen Roboterarm (BR) umfasst, der dazu bestimmt ist, mindestens ein chirurgisches Instrument zu tragen, wobei der Roboterarm (BR) ein Betätigungsmodul umfasst, das mindestens eine Betätigungsvorrichtung umfasst,
- eine Konsole (60), die eine Steuerschnittstelle (I) der Roboterplattform nach einem der vorstehenden Ansprüche umfasst,
- ein Behandlungsmodul (MT), das durch ein Kommunikationsnetzwerk mit der Steuerschnittstelle (I) und mit dem Betätigungsmodul verbunden ist, wobei das Behandlungsmodul (MT) mindestens einen Prozessor (52), einen Speicher (53) und eine Software umfasst, um die von der haptischen Vorrichtung (1) durchgeführten Messungen zu analysieren, und Bewegungsanweisungen zu berechnen und an den Roboterarm (BR) zu übergeben,
wobei die Plattform konfiguriert ist, sodass die auf den Stift (30) angewandten Bewegungen am chirurgischen Instrument anhand des Roboterarms (BR) reproduziert werden.

12. Robotersystem (SR) nach Anspruch 11, wenn von den Ansprüchen 2 bis 10 abhängig, wobei das Robotersystem (SR) konfiguriert ist, sodass die auf das Greiforgan (5) ausgeübten Drücke und die darauf angewandten Bewegungen durch das chirurgische Instrument anhand des Roboterarms (BR) reproduziert werden.

## Claims

1. A control interface (I) for a robotic platform for vitreoretinal surgery comprising a haptic device (1) equipped with an articulated chain (2) having a free end (3), **characterised in that** it comprises a guiding device (10) comprising:
- a stationary surface (11),
- a guiding means (20) mounted on the stationary surface by a ball joint (21),
- a rod (30) mounted on said guiding means (20) by a sliding connection (25) with an axis corresponding to that of the rod (30), said rod comprising a first end (32) mounted stationary on the free end (3) of the haptic device (1) and a second end (33) on which a gripping member (5) is intended to be mounted.

2. The control interface (I) according to claim 1, **characterised in that** it further comprises said gripping member (5), the gripping member (5) and the rod (30) respectively comprising detection means (6, 40) capable of detecting a pressure exerted by a user on said gripping member.

3. The control interface (I) according to claim 2, wherein a first detection means (6) is a magnet and a second detection means (40) is a Hall-effect sensor.

4. The control interface (I) according to claim 3, wherein the gripping member (5) comprises a deformable gripper (8) and a movable portion (7) on which said magnet (6) is mounted stationary, said movable portion (7) being able to displace when the gripper (8) deforms.

5. The control interface (I) according to any of claims 3 to 4, wherein the rod (30) is in the form of a hollow body internally delimiting a housing (34), said second detection means (40) being arranged in the housing (34).

6. The control interface (I) according to any one of the preceding claims, wherein the stationary surface (11) comprises an opening (12) and the guiding means (20) comprises a spherical member (23) and an element (22) for adapting the spherical member in the opening (12), said opening (12), the adapting element (22) and said spherical member (23) forming the ball joint (21).

7. The control interface (I) according to claim 6, wherein the rod (30) is cylindrical in shape, the spherical member (23) being hollow and having a plain bearing (24) matching the shape of the rod (30) so as to form the sliding connection (25).

8. The control interface (I) according to any one of claims 5 to 7, wherein the rod (30) comprises supply means (41) for the second detection means (40) arranged in the housing (34), said supply means (41) being electrically connected to the second detection means and able to receive an electrical signal (SE) emitted by the second detection means (40) when a pressure is exerted by a user on said gripping member (5).

9. The control interface (I) according to the preceding claim, wherein the guiding device (10) comprises a rotating collector (44) arranged coaxially around the rod (30), said collector (44) comprising an inner ring (44a) attached to the rod (30) and an outer ring (44b) attached to an axle of the articulated chain (2) on which the free end (3) of the haptic device (1) is located.

10. The control interface (I) according to any one of claims 2 to 9, comprising a sterile cover (15) interposed between the gripping member (5) and the second end (33) of the rod.

11. A robotic system (SR) for the vitreoretinal surgery comprising:
- a robotic platform (PR) comprising at least one robotic arm (BR) for carrying at least one surgical instrument, said robotic arm (BR) comprising an actuation module comprising at least one actuator,
- a console (60) comprising a control interface (I) for the robotic platform according to any of the preceding claims,
- a treatment module (MT) connected to the control interface (I) and to the actuation module by a communication network, said treatment module (MT) comprising at least one processor (52), a memory (53) and a software for analysing the measurements performed by the haptic device (1) and for calculating and giving movement setpoints to the robotic arm (BR),
said platform being configured so that the movements applied to the rod (30) are reproduced on said surgical instrument by means of the robotic arm (BR).

12. The robotic system (SR) according to claim 11 when dependent on claims 2 to 10, wherein the robotic system (SR) is configured such that the pressures exerted on the gripping member (5) and the movements which are applied to it are reproduced by the surgical instrument by means of the robotic arm (BR).
